# EUROPEAN PATENT APPLICATION

(11) **EP 3 305 799 A2**
(43) Date of publication of application: **11.04.2018**
(21) Application number: 17195298.9
(22) Date of filing: 06.10.2017
(51) Int. Cl.: C07J 9/00

(54) **SALTS OF OBETICHOLIC ACID**

(30) Priority: 07.10.2016 IN 201621034529
(71) Applicant: Lupin Limited, Mumbai, Maharashtra 400 055 (IN)
(72) Inventor: SANPHUI, Palash, 412 115 Pune (IN); SHIVDAVKAR, Radhakrishna Bhikaji, 412 115 Pune (IN); VYAS, Rajesh, 412 115 Pune (IN); BHISE, Nandu Baban, 412 115 Pune (IN); SINGH, Girij Pal, 412 115 Pune (IN)
(74) Representative: Chapman IP

(57) **Abstract**

The invention relates to salts of Obeticholic Acid, their amorphous and crystalline polymorphic form and processes for preparation thereof.

## Description

### Field of the invention

The present invention relates to salts of Obeticholic Acid, their amorphous and crystalline polymorphic forms and processes for preparation thereof.

### Background of the invention

Obeticholic Acid is useful for the treatment of primary biliary cholangitis (PBC) in combination with ursodeoxycholic acid (UDCA) in adults with an inadequate response to UDCA, or as a single therapy in adults unable to tolerate UDCA.

Obeticholic Acid is a farnesoid X receptor (FXR) agonist. It is a semi-synthetic bile acid analogue structurally represented in Figure-1 as given below:

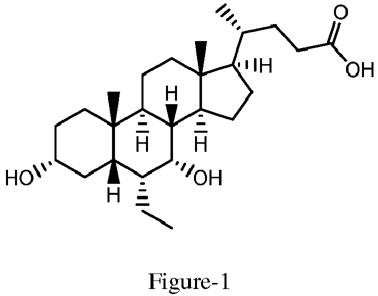

U.S. Patent No. 7,138,390 B2 discloses and claims Obeticholic Acid and pharmaceutically acceptable salts, solvates or amino acid conjugates thereof; specifically glycine and taurine conjugates.

WO2016044680 discloses salts of Obeticholic Acid and composition thereof. WO2017137931 discloses (S)-α-methyl benzyl amine and diethylamine salt of Obeticholic Acid.

Active pharmaceutical ingredients often do not exhibit the range of physical properties that makes them directly suitable for formulation development. One of the approaches that are used to modify the characteristics of drug substances is to employ a salt form of the substance. The beneficial aspects of using salt forms as active pharmaceutical ingredients are well known, and enable one to modify aqueous solubility, dissolution rate, solution pH, solid form, hygroscopicity, chemical stability, melting point, and even mechanical properties.

Salt formation is a relatively simple and powerful pre-formulation technique that can result in significant improvement of drug's physicochemical properties. Importantly, different salt forms rarely change drug's pharmacological properties.

Moreover introducing counter ions to the drug structure may result in the increased formation of solid forms, hydrates and solvates which ultimately leads to increase in variability of the drug's pharmaceutical properties providing broader scope to a formulation scientist for formulation optimization for example by providing a product with different properties, e.g., better processing or handling characteristics, improved dissolution profile, or improved shelf-life.

For these reasons, there is a need to study salts of Obeticholic Acid and their solid state characteristics.

### Object of the invention

It is an object of the present invention to provide salts of Obeticholic Acid and processes for preparation thereof.

It is another object of the present invention to provide salts of Obeticholic Acid with amino acids, ammonia, organic amines, alkali metals and the like and processes for preparation thereof.

It is another object of the present invention to provide amorphous and crystalline polymorphic forms of salts of Obeticholic Acid and processes for preparation thereof.

It is yet another object of the present invention to provide pharmaceutical composition comprising the salts of Obeticholic Acid.

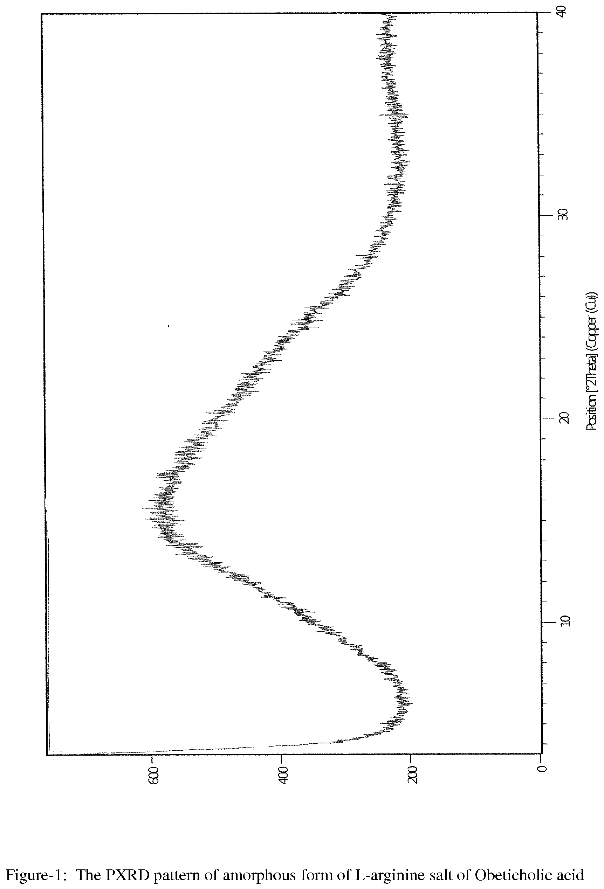

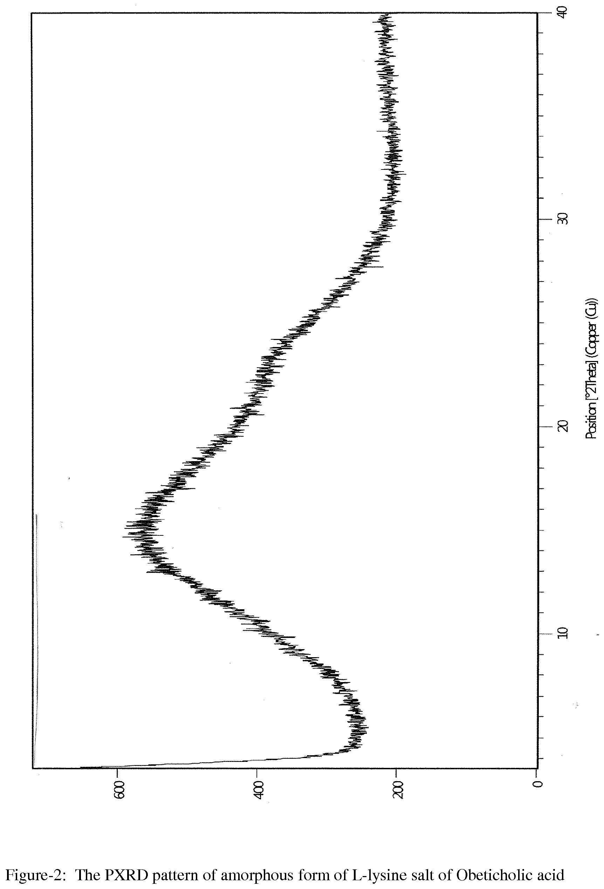

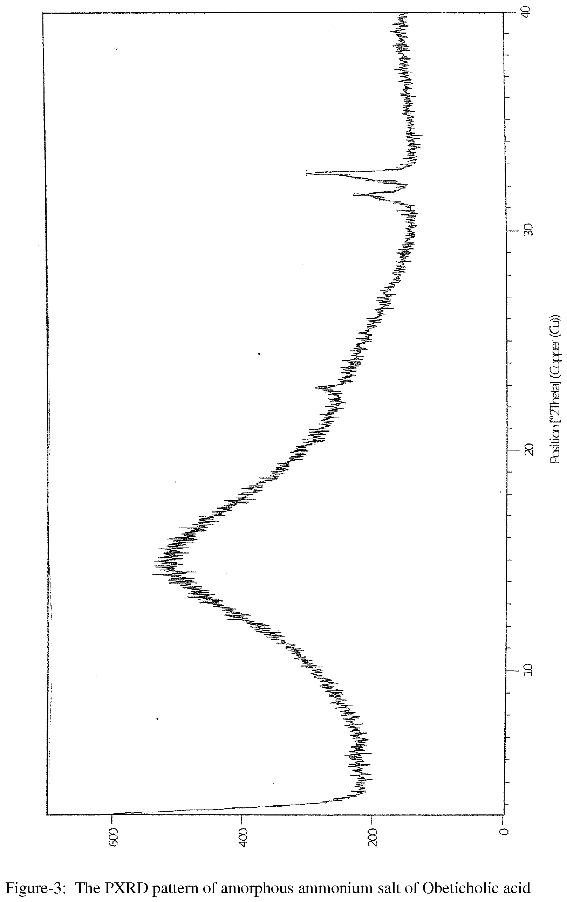

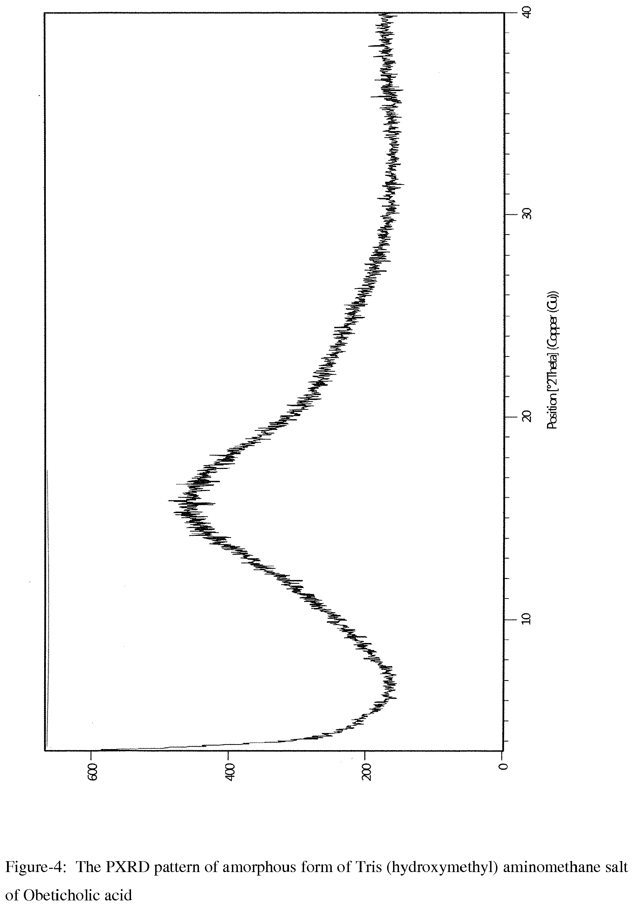

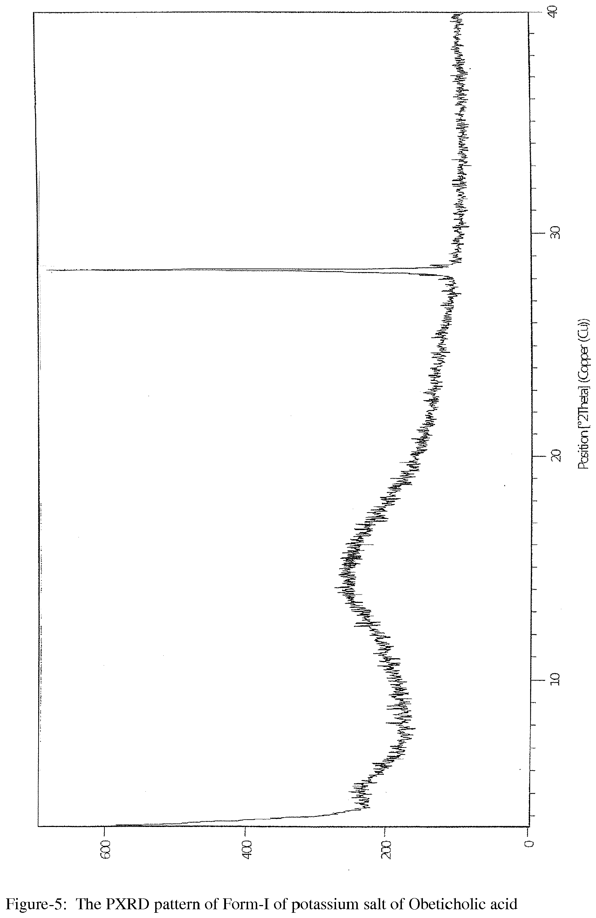

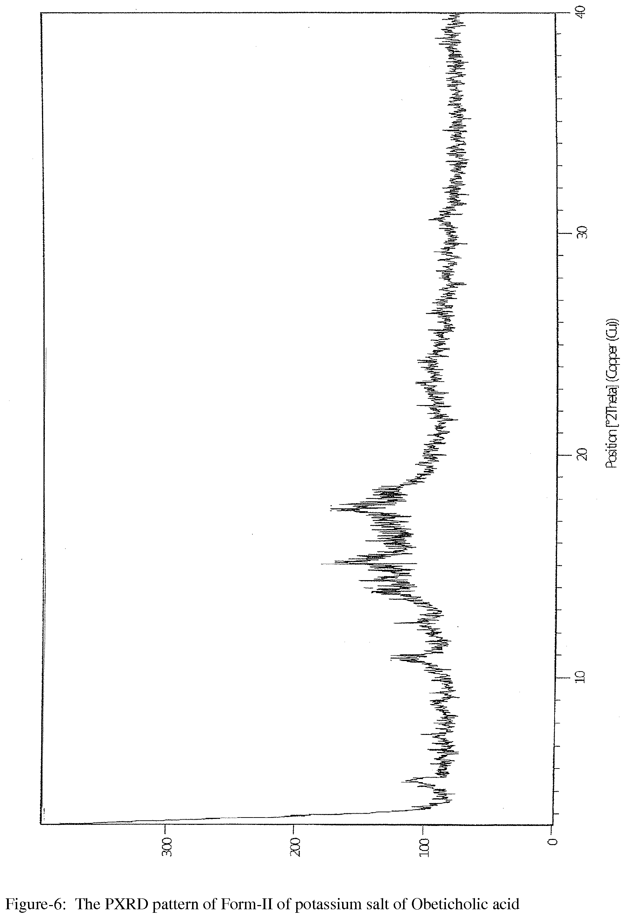

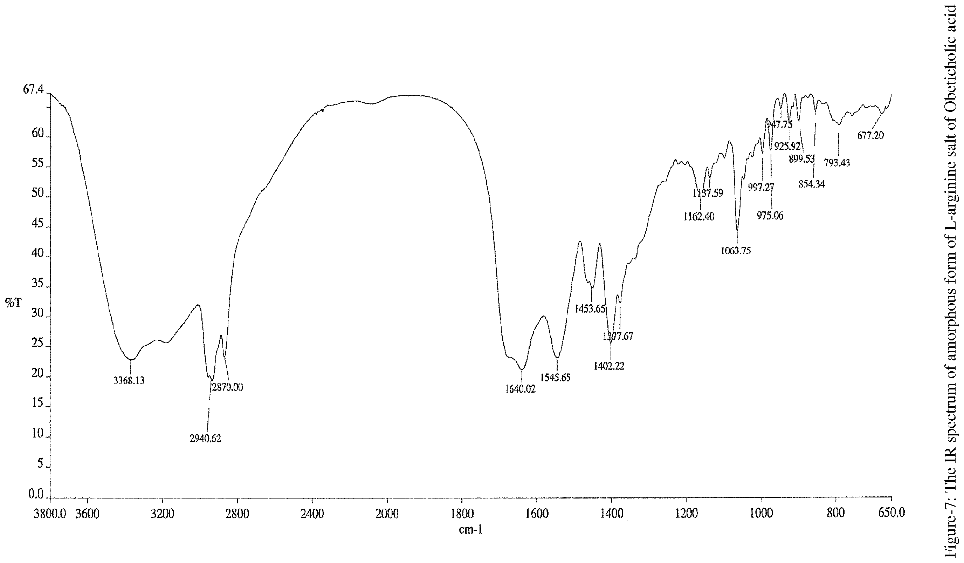

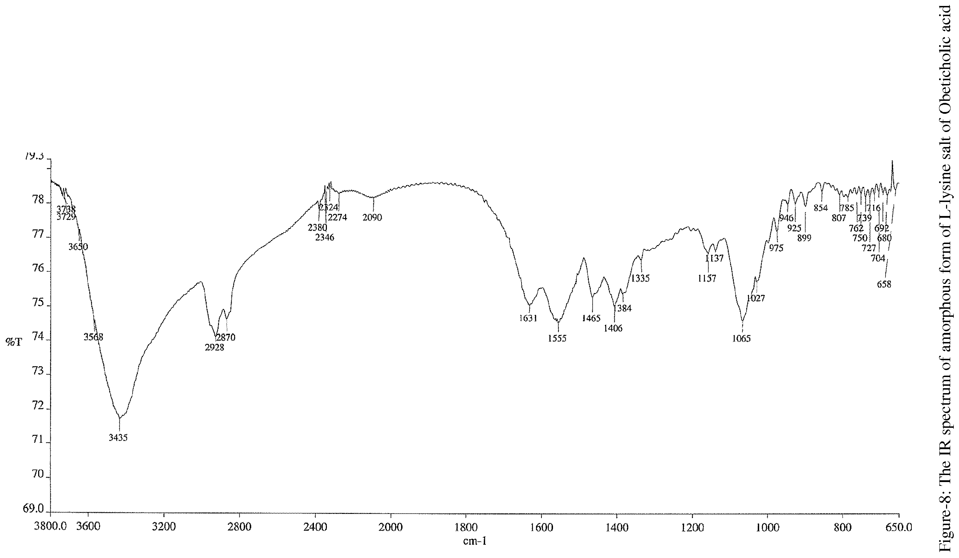

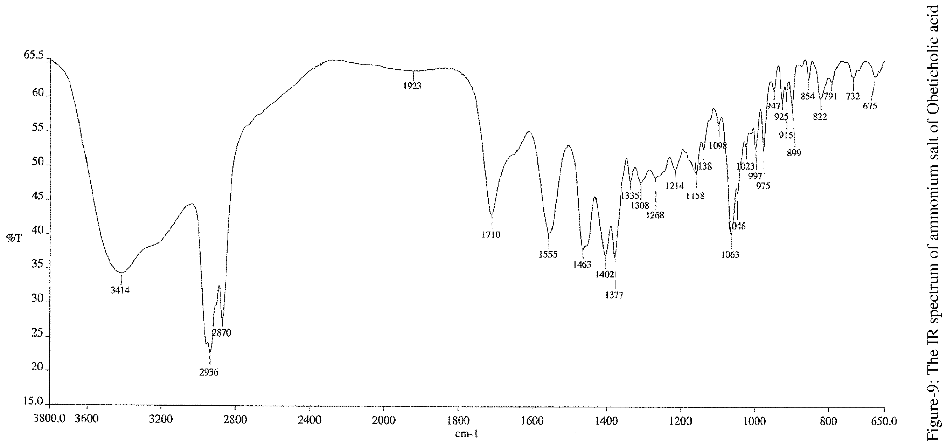

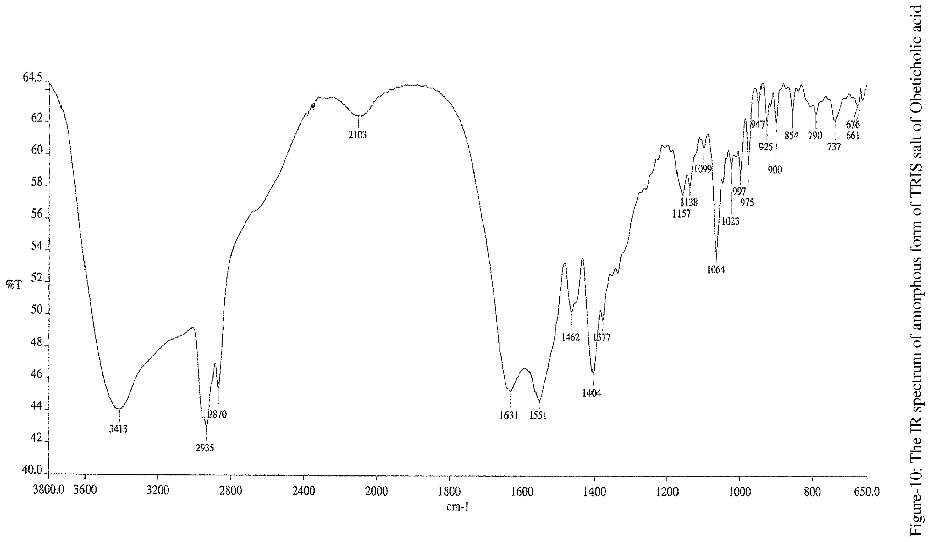

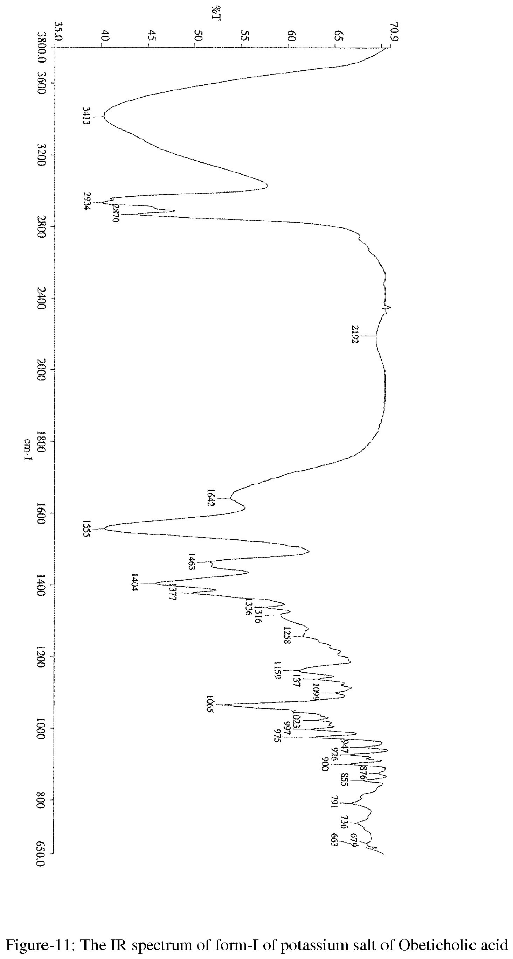

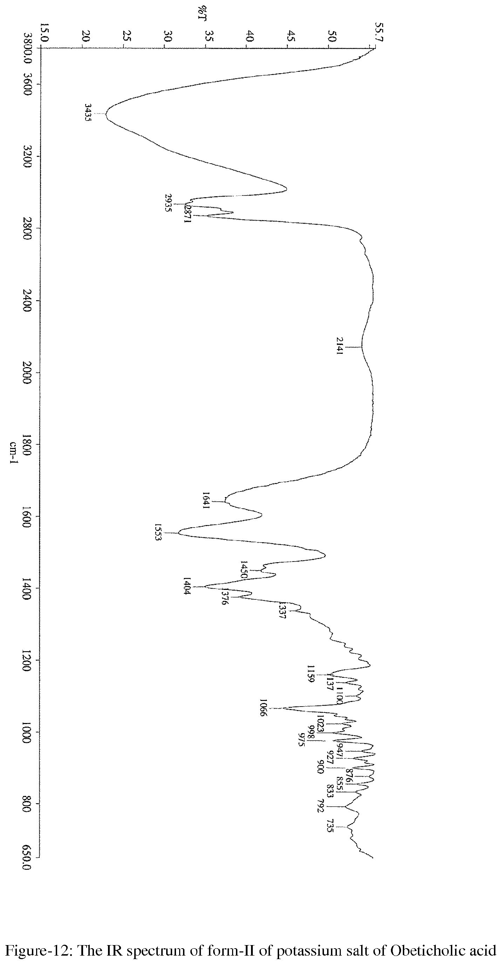

### Brief description of the drawing

Figure-1: The PXRD pattern of amorphous form of L-arginine salt of Obeticholic Acid
Figure-2: The PXRD pattern of amorphous form of L-lysine salt of Obeticholic Acid
Figure-3: The PXRD pattern of amorphous ammonium salt of Obeticholic Acid
Figure-4: The PXRD pattern of amorphous form of Tris (hydroxymethyl) aminomethane salt of Obeticholic Acid
Figure-5: The PXRD pattern of Form-I of potassium salt of Obeticholic Acid
Figure-6: The PXRD pattern of Form-II of potassium salt of Obeticholic Acid
Figure-7: The IR spectrum of amorphous form of L-arginine salt of Obeticholic Acid
Figure-8: The IR spectrum of amorphous form of L-lysine salt of Obeticholic Acid
Figure-9: The IR spectrum of amorphous form of ammonium salt of Obeticholic Acid
Figure-10: The IR spectrum of amorphous form of Tris (hydroxymethyl) aminomethane salt of Obeticholic Acid
Figure-11: The IR spectrum of Form-I of potassium salt of Obeticholic Acid
Figure-12: The IR spectrum of Form-II of potassium salt of Obeticholic Acid

### Description of the invention

As used herein, the term "amorphous" refers to a form of a compound that lacks a distinct crystalline structure and long range periodicity.

As used herein, the term "crystalline" refers to a unique ordered arrangement of structural units in the crystal lattice of the compound so that crystalline solids have rigid long range order. The structural units that constitute the crystal structure can be atoms, molecules, or ions.

As used herein, the term "polymorphic" refers to one of the crystalline forms of a compound or to a compound that has more than one crystalline form.

As used herein, unless otherwise indicated, the term "salt" includes salts, conjugates and complexes of Obeticholic Acid.

The present invention is directed to salts of Obeticholic Acid, their amorphous and crystalline polymorphic forms and processes for preparation thereof.

The present invention is directed to salts of Obeticholic Acid with amino acids, ammonia, organic amines, alkali metals and the like.

In an embodiment the present invention is directed to salts of Obeticholic Acid with D or L enantiomer of amino acids and their amorphous and crystalline polymorphic forms. Preferably, the amino acid is selected from the group consisting of alanine, asparagine, aspartic acid, arginine, glutamine, glycine, glutamic acid, histidine, isoleucine, lysine, leucine, phenylalanine, methionine, serine, proline, tryptophan, threonine, tyrosine and valine and the like.

In a preferred embodiment the present invention is directed to L-arginine and L-lysine salts of Obeticholic Acid. The present invention also provides a process for preparation of L-arginine and L-lysine salt of Obeticholic Acid.

The amino acid salt of Obeticholic Acid is prepared by taking Obeticholic Acid and the amino acid, specifically L-arginine and L-lysine, in a suitable solvent and heating to about 30 °C to about 90 °C, preferably to about 50 °C to about 80 °C followed by removal of the solvent to isolate the amino acid conjugate of Obeticholic Acid by a suitable technique.

The suitable solvent is selected from the group consisting of alcohol, ketone, chlorinated hydrocarbon, ester, nitrile, water or combinations thereof in a suitable proportion. The preferred alcohol is methanol, ethanol, propanol, isopropanol, butanol, 2-butanol, 1-pentanol and the like; the preferred ketone is acetone, methyl ethyl ketone and the like; the preferred chlorinated hydrocarbon is dichloromethane and the like; the preferred ester is ethyl acetate, isopropyl acetate and the like and the preferred nitrile is acetonitrile and the like.

Suitable techniques for solvent removal include slow evaporation, decantation, filtration by gravity or suction or centrifugation, using a rotational distillation device such as a Buchi® Rotavapor®, spray drying, agitated thin film drying, freeze drying (lyophilization), and the like, or any other suitable technique known in the art.

The isolation is done by methods known in the art, for example, solvent removal followed by trituration or recrystallization using a suitable solvent or addition of antisolvent.

In an embodiment, the present invention is directed to an amorphous form of L-arginine salt of Obeticholic Acid characterized by powder X-ray diffraction (PXRD) and Infrared (IR) spectroscopy.

The amorphous form of L-arginine salt of Obeticholic Acid is characterized by a PXRD pattern, substantially as illustrated by Figure-1.

The amorphous form of L-arginine salt of Obeticholic Acid is also characterised by IR Spectrum as illustrated by Figure-7.

The amorphous form of L-arginine salt of Obeticholic Acid is characterized by infrared spectrum (KBr) comprising one or more characteristic peaks selected from absorption bands at about 3368.13, 2940.62, 2870, 1640.02, 1545.65, 1453.65, 1402.22, 1377.67, 1162.40, 1137.59, and 1063.75cm⁻¹.

A process for preparing the L-arginine salt of Obeticholic Acid comprises of the following steps:
(a) providing Obeticholic Acid and L-arginine in water and a water miscible solvent to obtain a reaction mixture,
(b) heating the reaction mixture,
(c) removing the solvent, and
(d) isolating the L-arginine salt of Obeticholic Acid.

The water miscible solvent is selected from the group comprising of methanol, ethanol, isopropyl alcohol, n- propanol, tetrahydrofuran, acetone, acetonitrile and mixtures thereof.

In an embodiment, the present invention is directed to an amorphous form of L-lysine salt of Obeticholic Acid characterized by powder X-ray diffraction (PXRD) and Infrared (IR) spectroscopy.

The amorphous form of L-lysine salt of Obeticholic Acid is characterized by a PXRD pattern, substantially as illustrated by Figure-2.

The amorphous form of L-lysine salt of Obeticholic Acid is also characterised by IR Spectrum as illustrated by Figure-8.

The amorphous form of L-lysine salt of Obeticholic Acid is characterized by infrared spectrum (KBr) comprising one or more characteristic peaks selected from absorption bands at about 3435, 2928, 2870, 1631, 1555, 1465, 1406 and 1065 cm⁻¹.

A process for preparing the L-lysine salt of Obeticholic Acid comprises of the following steps:
(a)providing Obeticholic Acid and L-lysine in water and a water miscible solvent to obtain a reaction mixture,
(b)heating the reaction mixture,
(c)removing the solvent, and
(d)isolating the L-Lysine salt of Obeticholic Acid.

The water miscible solvent is selected from the group comprising of methanol, ethanol, isopropyl alcohol, n- propanol, tetrahydrofuran, acetone, acetonitrile and mixtures thereof.

The present invention also provides a process for preparation of L-arginine and L-lysine salt of Obeticholic Acid as illustrated in Example-1 and Example-2.

In yet another embodiment the invention is directed to ammonium salt of Obeticholic Acid and its amorphous and crystalline polymorphic forms.

The present invention also provides a process for preparation of ammonium salt of Obeticholic Acid.

The ammonium salt of Obeticholic Acid is prepared by taking Obeticholic Acid in a suitable solvent adding aqueous ammonia at room temperature or optionally heating to about 30 °C to about 60 °C, preferably to about 40 °C to about 50 °C followed by solvent removal to isolate the ammonium salt of Obeticholic Acid by a suitable technique.

The suitable solvent is selected from the group consisting of alcohol, ketone, chlorinated hydrocarbon, ester, nitrile, water or combinations thereof in a suitable proportion. The preferred alcohol is methanol, ethanol, propanol, isopropanol, butanol, 2-butanol, 1-pentanol and the like the, preferred ketone is acetone and methyl ethyl ketone and the like, the preferred chlorinated hydrocarbon is dichloromethane and the like, the preferred ester is ethyl acetate, isopropyl acetate and the like and the preferred nitrile is acetonitrile and the like.

Suitable techniques for solvent removal include slow evaporation, decantation, filtration by gravity or suction or centrifugation, using a rotational distillation device such as a Buchi® Rotavapor® or any other suitable technique known in the art.

The isolation is done by methods known in the art, for example, solvent removal followed by trituration or recrystallization using a suitable solvent or addition of antisolvent.

In an embodiment, the present invention is directed to amorphous form of ammonium salt of Obeticholic Acid characterized by powder X-ray diffraction (PXRD). The amorphous form of ammonium salt of Obeticholic Acid is characterized by a PXRD pattern, substantially as illustrated by Figure-3.

The amorphous form of ammonium salt of Obeticholic Acid is further characterized by a PXRD pattern comprising one or more broad diffuse halos with characteristic X-ray diffraction peaks at 22.8, 31.6 and 32.55 degree 2θ ± 0.2 degree 2θ

The amorphous form of ammonium salt of Obeticholic Acid is also characterised by IR Spectrum as illustrated by Figure-9.

The amorphous form of ammonium salt of Obeticholic Acid is further characterized by infrared spectrum (KBr) comprising one or more characteristic peaks selected from absorption bands at about 3414, 2936, 2870, 1710, 1555, 1463, 1402, 1377 and 1063 cm⁻¹.

A process for preparing the ammonium salt of Obeticholic Acid comprises of the following steps:
(a) providing Obeticholic Acid in a water miscible solvent and aqueous ammonia,
(b) removing the solvent, and
(c) isolating the ammonium salt of Obeticholic Acid.

The water miscible solvent is selected from the group comprising of methanol, ethanol, isopropyl alcohol, n- propanol, tetrahydrofuran, acetone, acetonitrile and mixtures thereof.

The present invention also provides a process for preparation of ammonium salt of Obeticholic Acid as illustrated in Example-3.

In yet another embodiment the invention is directed to organic amine salts of Obeticholic Acid and their amorphous and crystalline polymorphic forms.

The organic amine is selected from the group consisting of alkyl and aryl amine. Preferably the organic amine is selected from the group consisting of methylamine, dimethylamine, trimethylamine, ethylamine, diethylamine, triethylamine, ethanolamine, ethylenediamine, meglumine, Tris(hydroxymethyl)aminomethane, metformin, tetramethyl quaternary ammonium, tetraethyl quaternary ammonium or choline and the like.

In a preferred embodiment the present invention is directed to Tris (hydroxymethyl) aminomethane salt of Obeticholic Acid.

The present invention also provides a process for preparation of the organic amine salt of Obeticholic Acid.

The organic amine salt of Obeticholic Acid is prepared by taking Obeticholic Acid in a suitable solvent, adding organic amine or the corresponding buffer at room temperature or optionally heating to about 30 °C to about 60 °C, preferably to about 40 °C to about 50 °C, followed by solvent removal to isolate the organic amine salt of Obeticholic Acid by a suitable technique.

The suitable solvent is selected from the group consisting of alcohol, ketone, chlorinated hydrocarbon, ester, nitrile, water or combinations thereof in a suitable proportion. The preferred alcohol is methanol, ethanol, propanol, isopropanol, butanol, 2-butanol, 1-pentanol and the like the, preferred ketone is acetone and methyl ethyl ketone and the like ,the preferred chlorinated hydrocarbon is dichloromethane and the like, the preferred ester is ethyl acetate, isopropyl acetate and the like and the preferred nitrile is acetonitrile and the like.

Suitable techniques for solvent removal include slow evaporation decantation, filtration by gravity or suction or centrifugation, using a rotational distillation device such as a Buchi® Rotavapor®, spray drying, agitated thin film drying, freeze drying (lyophilization), and the like or any other suitable technique known in the art.

The isolation is done by methods known in the art, for example, solvent removal followed by trituration or recrystallization using a suitable solvent or by addition of antisolvent.

In an embodiment, the present invention is directed to an amorphous form of Tris(hydroxymethyl)aminomethane salt of Obeticholic Acid characterized by powder X-ray diffraction (PXRD).The amorphous form of Tris(hydroxymethyl)aminomethane salt of Obeticholic Acid is characterized by a PXRD pattern, substantially as illustrated by Figure-4.

The amorphous form of Tris(hydroxymethyl)aminomethane salt of Obeticholic Acid is also characterised by IR Spectrum as illustrated by Figure-10.

The amorphous form of Tris(hydroxymethyl)aminomethane salt of Obeticholic Acid is further characterized by infrared spectrum (KBr) comprising one or more characteristic peaks selected from absorption bands at about 3413, 2935, 2870, 1631, 1551, 1462, 1404 and 1064 cm⁻¹.

A process for preparing the Tris(hydroxymethyl)aminomethane salt of Obeticholic Acid comprises of the following steps:
(a) providing Obeticholic Acid and Tris(hydroxymethyl)aminomethane buffer in water and a water miscible solvent to obtain a reaction mixture,
(b) heating the reaction mixture of step (a)
(c) removing the solvent, and
(d) isolating the Tris(hydroxymethyl)aminomethane salt of Obeticholic Acid.

The water miscible solvent is selected from the group comprising of methanol, ethanol, isopropyl alcohol, n- propanol, tetrahydrofuran, acetone, acetonitrile and mixtures thereof.

The present invention also provides a process for preparation of Tris(hydroxymethyl)aminomethane salt of Obeticholic Acid as illustrated in Example-4.

In an embodiment the invention is directed to alkali metal salt of Obeticholic Acid and their amorphous and crystalline polymorphic forms.

The alkali metal salt is selected from the group consisting of sodium, potassium and the like.

In a preferred embodiment the present invention is directed to potassium salt of Obeticholic Acid and their amorphous and crystalline polymorphic forms.

The present invention also provides a process for preparation of the alkali metal salt of Obeticholic Acid.

The alkali metal salt of Obeticholic Acid is prepared by taking Obeticholic Acid in a suitable solvent, adding alkali metal hydroxide, optionally in a solvent, and the like at room temperature or optionally heating to about 30 °C to about 60 °C, preferably to about 40 °C to about 50 °C, followed by solvent removal to isolate the alkali metal salt of Obeticholic Acid by using a suitable technique.

The suitable solvent is selected from the group consisting of alcohol, ketone, chlorinated hydrocarbon, ester, nitrile, water or combinations thereof in a suitable proportion. The preferred alcohol is methanol, ethanol, propanol, isopropanol, butanol, 2-butanol, 1-pentanol and the like; the preferred ketone is acetone and methyl ethyl ketone and the like; the preferred chlorinated hydrocarbon is dichloromethane and the like; the preferred ester is ethyl acetate, isopropyl acetate and the like and the preferred nitrile is acetonitrile and the like.

Suitable techniques for solvent removal include slow evaporation decantation, filtration by gravity or suction or centrifugation, using a rotational distillation device such as a Buchi® Rotavapor®, spray drying, agitated thin film drying, freeze drying (lyophilization), and the like or any other suitable technique known in the art.

The isolation is done by methods known in the art, for example, solvent removal followed by trituration or recrystallization using a suitable solvent or addition of antisolvent.

In an embodiment, the present invention is directed to a Form I of potassium salt of Obeticholic Acid characterized by powder X-ray diffraction (PXRD).The Form I of potassium salt of Obeticholic Acid is characterized by a PXRD pattern, substantially as illustrated by Figure-5.

The Form I of potassium salt of Obeticholic Acid is characterized by a PXRD pattern comprising one or more broad diffuse halos with characteristic X-ray diffraction peak at 28.38 degree 2θ ± 0.2 degree 2θ.

The Form I of potassium salt of Obeticholic Acid is also characterised by IR Spectrum as illustrated by Figure-11.

The Form I of potassium salt of Obeticholic Acid is further characterized by infrared spectrum (KBr) comprising one or more characteristic peaks selected from absorption bands at about 3413, 2934, 2870, 1642, 1555, 1463, 1404, 1377, 1159 and 1065 cm⁻¹.

A process for preparing the Form I of potassium salt of Obeticholic Acid comprises of the following steps:
(a) providing Obeticholic Acid in a suitable solvent,
(b) contacting with potassium hydroxide in methanol,
(c) removing the solvent under vacuum, and
(d) isolating the Form I of potassium salt of Obeticholic Acid.

The suitable solvent of step (a) is selected from the group comprising of methanol, ethanol, isopropyl alcohol, n- propanol and mixtures thereof.

In yet another embodiment, the present invention is directed to a Form II of potassium salt of Obeticholic Acid characterized by powder X-ray diffraction (PXRD).The Form II of potassium salt of Obeticholic Acid is characterized by a PXRD pattern, substantially as illustrated by Figure-6.

The Form II of potassium salt of Obeticholic Acid is characterized by a PXRD pattern comprising of broad characteristic X-ray diffraction peaks at 5.4, 10.8, 13.8, 15.2 and 17.50 degree 2θ ± 0.2 degree 2θ.

The Form II of potassium salt of Obeticholic Acid is also characterised by IR Spectrum as illustrated by Figure-12.

The Form II of potassium salt of Obeticholic Acid is further characterized by infrared spectrum (KBr) comprising one or more characteristic peaks selected from absorption bands at about 3435, 2935, 2871, 1641, 1553, 1450, 1404, 1376, 1337, 1159 and 1066 cm⁻¹.

A process for preparing the Form II of potassium salt of Obeticholic Acid comprises of the following steps:
(a) providing Obeticholic Acid in a water miscible solvent,
(b) contacting with aqueous solution of potassium hydroxide
(b) removing the solvent under vacuum, and
(c) isolating the Form II of potassium salt of Obeticholic Acid.

The water miscible solvent is selected from the group comprising of methanol, ethanol, isopropyl alcohol, n- propanol, tetrahydrofuran, acetone, acetonitrile and mixtures thereof.

The present invention also provides a process for preparation of Form I of potassium salt of Obeticholic Acid and Form II of potassium salt of Obeticholic Acid as illustrated in Example-5 and Example-6.

In an embodiment the present invention also relates to process for preparation of non-crystalline Obeticholic Acid comprising converting the L-arginine salt, L-lysine salt, ammonium salt, Tris(hydroxymethyl)aminomethane and potassium salt to non-crystalline Obeticholic Acid using conventional techniques determined by one skilled in the art.

In one more embodiment, the present invention also provides a pharmaceutical composition comprising salts of Obeticholic Acid of the present invention along with one or more pharmaceutically acceptable carriers, excipients, or diluents.

The Obeticholic Acid salts and their amorphous and crystalline polymorphic forms provided by the present invention may be used as FXR agonist indicated for the treatment of primary biliary cholangitis (PBC). Such pharmaceutical composition can be prepared by the methods known in the literature.

The present invention is further illustrated with the following non-limiting examples.

### Example-1: Preparation of amorphous L-arginine salt of Obeticholic Acid

To a mixture of 1.0 gm of Obeticholic Acid and 0.42 gm of L-arginine, 20 mL of methanol was added and heated to 50°C followed by addition of 10 mL of water and stirring at 50°C for 5 to 10 min. to obtain a clear solution. The clear reaction mass was filtered followed by solvent removal under vacuum to yield 1.2 gm of amorphous L-arginine salt of Obeticholic Acid.

### Example-2: Preparation of amorphous L-lysine salt of Obeticholic Acid

To a mixture of 2.0 gm of Obeticholic Acid and 0.7 gm of L-lysine, 30 mL of methanol was added and heated to 50°C followed by addition of 8 mL of water and stirring at 50°C for 5 to10 min. The reaction mass was filtered followed by solvent removal under vacuum to yield 2.1 gm of amorphous L-lysine salt of Obeticholic Acid.

### Example-3: Preparation of ammonium salt of Obeticholic Acid

To a mixture of 2.0 gm of Obeticholic Acid and 20 mL methanol, 0.9 gm aqueous ammonia was added followed by solvent removal under vacuum to yield 1.8 gm of ammonium salt of Obeticholic Acid.

### Example-4: Preparation of amorphous form of Tris(hydroxymethyl)aminomethane salt of Obeticholic Acid

To a mixture of 2.0 gm of Obeticholic Acid and 0.6 gm of Tris(hydroxymethyl)aminomethane buffer, 20 mL of methanol was added and stirred for 5 to 10 min. Thereafter the reaction mixture was heated to 50°C followed by addition of 4 mL water. The clear reaction mass was filtered and left overnight for slow evaporation of solvent to yield a sticky mass. The sticky mass was dried under vacuum to yield 1.9 gm of amorphous form of Tris(hydroxymethyl)aminomethane salt of Obeticholic Acid.

### Example-5: Preparation of Form I of potassium salt of Obeticholic Acid

To a mixture of 2.0 gm of Obeticholic Acid and 20 mL of methanol was added 0.28 gm of potassium hydroxide pellets in 20 mL methanol and stirred. Thereafter solvent was removed under vacuum to yield 1.9 gm of Form I of potassium salt of Obeticholic Acid.

### Example-6: Preparation of Form II of potassium salt of Obeticholic Acid

To 3.0 gm of Obeticholic Acid, 40 mL of acetone was added and stirred at 25°C to 30°C to get a clear reaction mass, followed by addition of a solution of 0.5g potassium hydroxide in 1.0 mL water. The reaction mass was diluted with 10.0 ml more of acetone, cooled to -10 to -2 °C and stirred for 30 min. Raised temperature to 15 to 20 °C and continued stirring for 60 min. followed by filtration and drying under vacuum to yield 2.3 gm of Form II of potassium salt of Obeticholic Acid.

## Claims

1. L-arginine salt of Obeticholic Acid.

2. A process for preparing L-arginine salt of Obeticholic Acid of claim 1, comprising the steps of:
(a) providing Obeticholic Acid and L-Arginine in water and a water miscible solvent to obtain a reaction mixture,
(b) heating the reaction mixture,
(c) removing the solvent, and
(d) isolating the L-arginine salt of Obeticholic Acid.

3. The process according to claim 2 wherein the water miscible solvent is selected from the group comprising of methanol, ethanol, tetrahydrofuran, acetone and acetonitrile.

4. L-lysine salt of Obeticholic Acid.

5. A process for preparing L-lysine salt of Obeticholic Acid of claim 4, comprising the steps of:
(a) providing Obeticholic Acid and L-lysine in water and a water miscible solvent to obtain a reaction mixture,
(b) heating the reaction mixture,
(c) removing the solvent, and
(d) isolating the L-arginine salt of Obeticholic Acid.

6. The process according to claim 5 wherein the water miscible solvent is selected from the group comprising of methanol, ethanol, tetrahydrofuran, acetone and acetonitrile.

7. Ammonium salt of Obeticholic Acid.

8. A process for preparing the ammonium salt of Obeticholic Acid of claim 7 comprising the steps of:
(a) providing Obeticholic Acid in a water miscible solvent and aqueous ammonia,
(b) removing the solvent, and
(c) isolating the ammonium salt of Obeticholic Acid.

9. The process according to claim 8 wherein the water miscible solvent is selected from the group comprising of methanol, ethanol, tetrahydrofuran, acetone and acetonitrile.

10. Tris (hydroxymethyl) aminomethane salt of Obeticholic Acid.

11. A process for preparing the Tris (hydroxymethyl) aminomethane salt of Obeticholic Acid of claim 10, comprising the steps of:
(a) providing Obeticholic Acid and Tris(hydroxymethyl)aminomethane buffer in water and a water miscible solvent to obtain a reaction mixture,
(b) heating the reaction mixture of step (a),
(c) removing the solvent, and
(d) isolating the Tris(hydroxymethyl)aminomethane salt of Obeticholic Acid.

12. The process according to claim 11 wherein the water miscible solvent is selected from the group comprising of methanol, ethanol, tetrahydrofuran, acetone and acetonitrile.

13. Form-I of potassium salt of Obeticholic Acid.

14. The Form-I of potassium salt of Obeticholic Acid of claim 13 **characterized by** a powder X-ray diffraction pattern substantially as depicted in Figure-5.

15. A process for preparing the Form I of potassium salt of Obeticholic Acid of claim 13 comprising the steps of:
(a) providing Obeticholic Acid in a suitable solvent,
(b) contacting with potassium hydroxide in methanol,
(c) removing the solvent under vacuum, and
(d) isolating the Form I of potassium salt of Obeticholic Acid.

16. The process according to claim 15 wherein the suitable solvent is selected from the group comprising of methanol, ethanol, isopropanol and n-propanol.

17. Form-II of potassium salt of Obeticholic Acid.

18. The Form-II of potassium salt of Obeticholic Acid of claim 17 **characterized by** a powder X-ray diffraction pattern substantially as depicted in Figure-6.

19. A process for preparing the Form II of potassium salt of Obeticholic Acid of claim 17 comprising the steps of:
(a) providing Obeticholic Acid in a water miscible solvent,
(b) contacting with aqueous solution of potassium hydroxide
(c) removing the solvent under vacuum, and
(d) isolating the Form II of potassium salt of Obeticholic Acid.

20. The process according to claim 19 wherein the water miscible solvent is selected from the group comprising of methanol, ethanol and isopropanol.
